(19) Europäisches Patentamt
European Patent Office
Office européen des brevets

(11) Publication number: **0 085 870**
**B1**

(12) **EUROPEAN PATENT SPECIFICATION**

(45) Date of publication of patent specification: **11.06.86**

(51) Int. Cl.[4]: **C 07 D 313/12**, A 61 K 31/335

(21) Application number: **83100533.5**

(22) Date of filing: **21.01.83**

(54) **Dibenz(b,e)oxepin derivatives and pharmaceutical compositions containing them.**

(30) Priority: **25.01.82 JP 9843/82**

(43) Date of publication of application:
**17.08.83 Bulletin 83/33**

(45) Publication of the grant of the patent:
**11.06.86 Bulletin 86/24**

(84) Designated Contracting States:
**DE FR GB**

(56) References cited:
**EP-A-0 038 564**
**US-A-4 282 365**

**EUROPEAN JOURNAL OF MEDICAL CHEMISTRY - CHIMICA THERAPEUTICA, vol. 9, no. 3, 1974, P. DOSTERT et al. "Composes tricycliques portant une chaine alkylaminoalkylthio. Synthese et activite pharmacologique"**

(73) Proprietor: **KYOWA HAKKO KOGYO CO., LTD**
**Ohtemachi Bldg. Ohtemachi 1-chome Chiyoda-ku**
**Tokyo (JP)**

(72) Inventor: **Takizawa, Hiroshi**
**2-18-35, Kobara-cho**
**Mishima-shi Shizuoka-ken (JP)**
Inventor: **Morita, Osamu**
**410-1, Nameri Nagaizumi-cho**
**Sunto-gun Shizuoka-ken (JP)**
Inventor: **Oiji, Yoshimasa**
**69-5, Shimonagakubo Nagaizumi-cho**
**Sunto-gun Shizuoka-ken (JP)**
Inventor: **Hashimoto, Tamotsu**
**3592-11, Jinba Aza Ohoka**
**Numazu-shi Shizuoka-ken (JP)**

(74) Representative: **Casalonga, Axel et al**
**BUREAU D.A. CASALONGA OFFICE JOSSE & PETIT Morassistrasse 8**
**D-8000 München 5 (DE)**

**EP 0 085 870 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to novel dibenz[b,e]oxepin derivatives, the pharmaceutically acceptable and addition salts thereof and a pharmaceutical composition containing, as the active ingredient, a dibenz[b,e]oxepin derivative.

More particularly, the present invention pertains to a novel dibenz[b,e]oxepin derivative represented by the following general formula (Ia):

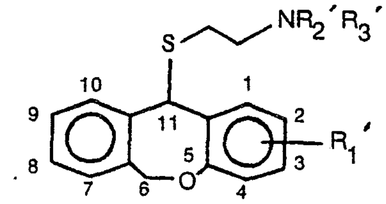

(Ia)

wherein $R_1'$ represents an alkyl group having 1 to 5 carbon atoms or a halogen atom, $R_2'$ and $R_3'$ may be same or different group and each represents an alkyl group having 1 to 5 carbon atoms; provided that when $R_2'$ and when $R_3'$ each represents a methyl group, $R_1'$ does not represent a methyl group, a fluorine atom, a chlorine atom or a bromine atom; and the pharmaceutically acceptable acid addition salts thereof, and to pharmaceutical compositions containing them.

In addition, the present invention pertains to the use of a dibenz[b,e]oxepin derivative represented by the following general formula (I)

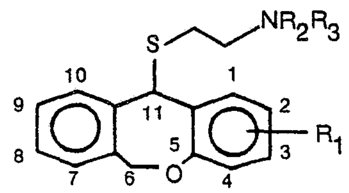

(I)

wherein $R_1$ represents an alkyl group having 1 to 5 carbon atoms or a halogen atom, $R_2$ and $R_3$ may be same or different group and each represents an alkyl group having 1 to 5 carbon atoms; and the pharmaceutically acceptable acid addition salts thereof, for the manufacture of an antiasthmatic medicament.

A dibenz[b,e]oxepin derivative of the present invention and the pharmaceutically acceptable acid addition salts thereof have antiasthmatic activity and are therefore useful as an antiasthmatic agent.

Among the compounds represented by the general formula (I), compounds wherein $R_2$ and $R_3$ represent a methyl group and $R_1$ represents a methyl group, a fluorine atom, a chlorine atom or a bromine atom are known compounds which are described in *Eur. J. Med. Chem. Chimica Therapeutica 9,* 259 (1974). While it is described in the reference that such compounds have antidepressant and peripheral anticholinergic activities, it is not disclosed or suggested that the compounds have an antiasthmatic activity.

The present inventors have now found that compound I has an antiasthmatic activity. The present invention is described in detail below.

In the definition of $R_1'$, $R_2'$ and $R_3'$ in the general formula (Ia) and $R_1$, $R_2$ and $R_3$ in the general formula (I), the alkyl group having 1 to 5 carbon atoms includes a methyl group, an ethyl group, a propyl group, a butyl group, an amyl group, etc.; and the halogen atom includes a fluorine atom, a chlorine atom, a bromine atom, and an iodine atom.

The acid addition salts of the general formulae (Ia) and (I) include inorganic acid addition salts such as hydrochloride, sulfate, hydrobromide, phosphate, etc. and organic acid addition salts such as acetate, maleate, fumarate, tartrate, citrate, oxalate, benzoate, etc.

A process for production of a compound represented by the general formula (I) (hereinafter referred to as compound I) is shown below.

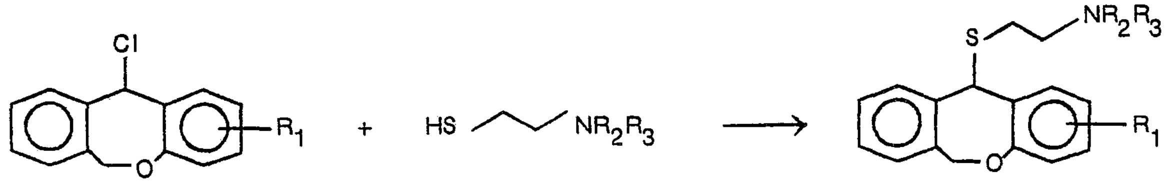

Compound II Compound III Compound I

wherein $R_1$, $R_2$ and $R_3$ have the same meanings as defined above.

Equimolecular quantities of compound II and compound III (or an acid addition salt of compound III) are dissolved in an inert solvent such as chloroform, methylene chloride, toluene, tetrahydrofuran, N,N-dimethylformamide, etc. and the mixture is stirred at room temperature to the boiling point of the inert solvent used for 30 minutes to 2 hours. Then, the solvent is removed from the reaction solution to obtain a crude salt of compound I as a residue.

The crude product is dissolved in an aqueous basic solution, and then the mixture is extracted with an organic solvent difficulty miscible with water such as diethylether, whereby the desired compound is obtained in the form of free base. Because of unlikeliness to crystallize, the product, if necessary, is subjected to purification by column chromatography, etc. and then, an appropriate acid is added thereto to obtain an acid addition salt thereof, which is more tractable. Further if necessary, the acid addition salt may be converted to a highly-purified product by a suitable recrystallization operation. It goes without saying, that the aforesaid residual crude product after removal of the reaction solvent is immediately subjected to a recrystallization treatment without the liberation process whereby a purified product is obtained.

As an appropriate acid, a physiologically usable inorganic acid such as hydrochloric acid, sulfuric acid, hydrobromic acid and phosphoric acid or an organic acid such as acetic acid, maleic acid, fumaric acid, tartaric acid, citric acid, oxalic acid and benzoic acid may be used. Compound II, a starting material for compound I is a known compound which is disclosed in Japanese Published Unexamined Patent Application Nos. 150082/81 and 150083/81, and compound III is on the market and readily available. Results of acute toxicity test and antiasthmatic activity test of compound I are shown below.

Acute toxicity test

Groups of male dd-strain mice (each group consisting of five mice) weighing 20 ± 1 g are used. Compound I are administered orally (po: 0.3 mg/g) or intraperitoneally (ip: 0.1 mg/g). The MLD (minimum lethal dose) is calculated from the mortality for 7 days after the administration to obtain the results given in Table 1.

TABLE 1

| Compound | MLD (mg/Kg) | |
|---|---|---|
| | po | ip |
| Compound 1 | > 300 | > 100 |
| ,, 2 | > 300 | > 100 |
| ,, 3 | > 300 | > 100 |
| ,, 4 | > 300 | > 100 |
| ,, 5 | > 300 | 100 |
| ,, 6 | > 300 | > 100 |
| ,, 7 | > 300 | > 100 |
| ,, 8 | > 300 | > 100 |
| ,, 9 | > 300 | > 100 |
| ,, 10 | > 300 | > 100 |
| ,, 11 | > 300 | > 100 |

Typical examples (compounds 1—11) of compound I are designated as follows.

| Compound | Name of compound |
|---|---|
| 1 | 2-methyl-11-[2-(dimethylamino)ethyl]thio-6,11-dihydrodibenz[b,e]oxepin |
| 2 | 2-methyl-11-[2-(diethylamino)ethyl]thio-6,11-dihydrodibenz[b,e]oxepin |
| 3 | 4-methyl-11-[2-(diethylamino)ethyl]thio-6,11-dihydrodibenz[b,e]oxepin |
| 4 | 2-chloro-11-[2-(diethylamino)ethyl]thio-6,11-dihydrodibenz[b,e]oxepin |
| 5 | 2-ethyl-11-[2-(diethylamino)ethyl]thio-6,11-dihydrodibenz[b,e]oxepin |
| 6 | 2-fluoro-11-[2-(diethylamino)ethyl]thio-6,11-dihydrodibenz[b,e]oxepin |
| 7 | 2-ethyl-11-[2-(dimethylamino)ethyl]thio-6,11-dihydrodibenz[b,e]oxepin |
| 8 | 2-fluoro-11-[2-(dimethylamino)ethyl]thio-6,11-dihydrodibenz[b,e]oxepin |
| 9 | 2-ethyl-11-[2-(dissopropylamino)ethyl]thio-6,11-dihydrodibenz[b,e]oxepin |
| 10 | 2-fluoro-11-[2-(diisopropylamino)ethyl]thio-6,11-dihydrodibenz[b,e]oxepin |
| 11 | 3-methyl-11-[2-(dimethylamino)ethyl]thio-6,11-dihydrodibenz[b,e]oxepin |

Antiasthmatic activity test
[Experimental method]

Antiasthmatic activity is evaluated according to passive cutaneous anaphylaxis response (PCA response). Antiserum is collected from Wistar strain male rats weighing 200 to 250 g. PCA response is evaluated using Wistar strain male rats weighing 100 to 120 g.

(A) Preparation of antiserum:

One mg of egg albumin is dissolved in 0.5 ml of pertussisdiphtheria mixed vaccine, and the resulting solution is mixed with 0.5 ml of incomplete adjuvant. The resulting emulsion is used as antigen and administered to rats subcutaneously through the sole. On the 12th day after the administration, heads of the rats are cut to collect blood. Thus, antiserum is prepared.

Upon evaluating antiasthmatic activity, concentration of the antiserum is adjusted so that diameter of blue-dyed portion is about 8 to about 10 mm.

(B) PCA response (evaluation of antiasthmatic activity):

Six rats are used per group. 0.05 ml of the antiserum is intracutaneously administered to each of the previously back-shaved rats to sensitize. After 17 hours, compound I or the solutions thereof (physiological salt solution or CMC solution) are respectively administered and, after 60 minutes, an antigen mixture (1% Evans' Blue physiological salt solution containing 0.2% egg albumin) is injected intravenously in an amount of 0.5 ml/100 g to induce PCA response. After 30 minutes, the rats are choked to death followed by cutting out skin to measure the diameter of blue-dyed portion. The results are evaluated by awarding a score according to the diameter of blue-dyed portion. Furthermore, ratios of the diameters to that of solvent (physiological salt solution or CMC) administered group are determined. Compounds showing a depressing ratio of 50% or more calculated according to the following formula are concluded to show positive antiasthmatic activity. Also, the minimum effective dose (MED) is determined from the results with respective doses to compare the strength of antiasthmatic activity. Results thus obtained are tabulated in Table 2.

| Score | Blue-dyed Portion ($\phi$ mm) |
|---|---|
| 5 | $\geq$ 10 mm |
| 4 | 8.0 — 9.9 mm |
| 3 | 6.0 — 7.9 ,, |
| 2 | 4.0 — 5.9 ,, |
| 1 | 2.0 — 3.9 ,, |
| 0 | 9 — 1.9 ,, |

Depressing ratio (%) =

$$\frac{\text{Diameter for solvent-administered group} - \text{Diameter for test compound-administered group}}{\text{Diameter for solvent-administered group}} \times 100$$

TABLE 2

| Compound \ Dose mg/Kg PO | 100 | 50 | 25 | 10 | 5 | 2.5 | 1 | MED mg/KgPO |
|---|---|---|---|---|---|---|---|---|
| Compound 1 | 100 | 100 | 100 | 100 | 100 | 81 | 49 | 2.5 |
| ,, 2 | 100 | 100 | 100 | 52 | 40 | 20 | — | 10.0 |
| ,, 3 | 52 | 14 | — | — | — | — | — | 100.0 |
| ,, 4 | 100 | 100 | 46 | 10 | — | — | — | 50.0 |
| ,, 5 | 100 | 100 | 100 | 56 | 36 | — | — | 10.0 |
| ,, 6 | 100 | 100 | 84 | 3 | — | — | — | 25.0 |
| ,, 7 | 100 | 100 | 100 | 100 | 81 | 30 | — | 5.0 |
| ,, 8 | 100 | 100 | 100 | 33 | 23 | — | — | 25.0 |
| ,, 9 | 100 | 100 | 67 | 22 | — | — | — | 25.0 |
| ,, 10 | 73 | 11 | 11 | 0 | — | — | — | 100.0 |
| ,, 11 | 61 | 61 | 28 | 0 | — | — | — | 50.0 |

As is apparent from Table 2, compound I has an antiasthmatic activity and is useful as an antiasthmatic agent.

Compound I may be used in various pharmaceutical forms for administration. Pharmaceutical compositions of the present invention are prepared by uniformly mixing an effective amount of compound I as the active ingredient, in free form or as an acid addition salt, with a pharmaceutically acceptable carrier.

The carrier may take various forms depending on the pharmaceutical form suitable for administration. It is preferable that the pharmaceutical composition is in single administration form suitable for administration orally or by injection.

To prepare the compositions of the present invention for oral administration, any useful pharmaceutical carrier may be used. For example, oral liquid preparations such as suspensions and syrups can be prepared using water, sugar (e.g. sucrose, sorbitol and fructose), glycols (e.g. polyethyleneglycol and propyleneglycol), oils (e.g. sesame oil, olive oil and soybean oil), antisepics (e.g. an alkyl parahydroxybenzoate), flavours (e.g. strawberry flavour and peppermint) and the like. Powders, pills, capsules and tablets can be prepared using excipients (e.g. lactose, glucose, sucrose and mannitol), disintegrators (e.g. starch and sodium alginate), lubricants (e.g. magnesium stearate and talc), binders (e.g. polyvinyl alcohol, hydroxypropylcellulose and gelatin), surfactants (e.g. sucrose fatty acid ester), plasticizers (e.g. glycerin) and the like.

Tablets and capsules are the most useful single oral administration forms because of the ease of administration. To make tablets and capsules solid pharmaceutical carriers are used.

An injection solution can be prepared using a carrier consisting of salt solution, glucose solution and a mixture of salt and glucose solution.

Although the amount of the active ingredient can be varied over a rather wide range, 1—20 mg/kg/day in one dose or several divided doses is generally considered to be effective.

Certain specific embodiments of the invention are illustrated by the following representative examples.

### Example 1

In this example, 1.70 g of diethylaminoethylthiolhydrochloride and 2.45 g of 2-methyl-11-chloro-6,11-dihydrodibenz[b,e]oxepin are dissolved in 50 ml of methylene chloride, and the mixture is stirred for 2 hours. The reaction solution is concentrated under reduced pressure to distill off methylene chloride. Recrystallization of the residue from 50 ml of ethanol gives 3.25 g of pure crystals of hydrochloride of 2-methyl-11-[2-(diethylamino)ethyl]thio-6,11-dihydrodibenz[b,e]oxepin (compound 2) in a yield of 86%.

m.p.: (hydrochloride) 202—205°C

IR absorption spectrum: (KBr tablet, $cm^{-1}$) 2920, 2670, 1495, 1260, 1230, 1015.

Elemental analysis of hydrochloride:

| | C(%) | H(%) | N(%) |
|---|---|---|---|
| Calcd. for $C_{21}H_{27}NOS{\cdot}HCl$: | 66.73 | 7.47 | 3.71 |
| Found: | 66.52 | 7.23 | 3.90 |

The resulting hydrochloride is dissolved in a basic water, and then the solution is extracted with diethylether. The extract is dehydrated and concentrated to obtain an oily free base.

NMR spectum ($CDCl_3$, δvalue, ppm): 0.93 (t, 6H), 2.22 (s, 3H), 1.93—2.79 (m, 8H), 4.77 (d, 1H), 4.89 (s, 1H), 6.23 (d, 1H), 6.52—7.45 (m, 7H).

### Examples 2 to 11

Compounds 1 and 3 through 11 having the physico-chemical properties identified in Tables 4 and 5 are obtained in a similar manner to that in Example 1 except that the aminoethylthiolhydrochloride and 11-chloro-6,11-dihydrobenz[b,e]oxepin shown in Table 3 are used instead of diethylaminoethylthiolhydrochloride and 2-methyl-11-chloro-6,11-dihydrodibenz[b,e]oxepin in Example 1.

TABLE 3

| Example | Aminoethylthiolhydrochloride 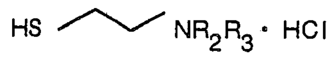 | | Used amount (g) |
|---|---|---|---|
| | $R_2$ | $R_3$ | |
| 2 | methyl | methyl | 1.4 |
| 3 | ethyl | ethyl | 1.0 |
| 4 | ethyl | ethyl | 1.4 |
| 5 | ethyl | ethyl | 1.7 |
| 6 | ethyl | ethyl | 1.7 |
| 7 | methyl | methyl | 1.4 |
| 8 | methyl | methyl | 1.4 |
| 9 | isopropyl | isopropyl | 2.0 |
| 10 | isopropyl | isopropyl | 2.0 |
| 11 | methyl | methyl | 2.8 |

TABLE 3 (Continued)

| Example | 11-chloro-6,11-dihydrodibenz [b,e] oxepin 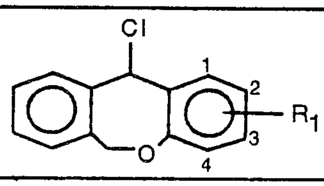 | Used amount (g) |
|---|---|---|
| | $R_1$ | |
| 2 | 2-methyl | 2.4 |
| 3 | 4-methyl | 1.4 |
| 4 | 2-chloro | 2.2 |
| 5 | 2-ethyl | 2.6 |
| 6 | 2-fluoro | 2.5 |
| 7 | 2-ethyl | 2.6 |
| 8 | 2-fluoro | 2.5 |
| 9 | 2-ethyl | 2.6 |
| 10 | 2-fluoro | 2.5 |
| 11 | 3-methyl | 5.2 |

TABLE 4

| Example | Compound | Obtained amount of the desired compound (g) | Yield (%) | Physicochemical properties | |
|---|---|---|---|---|---|
| | | | | m.p. (°C) (hydrochloride) | IR absorption spectrum (hydrochloride, KBr tablet, $cm^{-1}$) |
| 2 | Compound 1 | 1.8 | 51 | 166—169 | 2690, 1500, 1460, 1260, 1230, 1015 |
| 3 | Compound 3 | 2.1 | 97 | 163—165 | 2920, 2580, 2470, 1470, 1195, 1010 |
| 4 | Compound 4 | 3.1 | 93 | 165—167 | 2920, 2620, 1485, 1255, 1230, 1020 |
| 5 | Compound 5 | 3.6 | 90 | 157—160 | 2920, 2670, 1500, 1255, 1235, 1010 |
| 6 | Compound 6 | 3.6 | 94 | 134—137 | 2920, 2620, 1495, 1255, 1210, 1025 |
| 7 | Compound 7 | 2.9 | 80 | 149—151 | 2920, 1505, 1260, 1230, 1125, 1020 |
| 8 | Compound 8 | 3.2 | 90 | 169—172 | 2920, 2720, 1500, 1260, 1230, 1025 |
| 9 | Compound 9 | 3.8 | 98 | hydrochloride being unable to crystal- lize, and fumarate being too hygro- scopic to measure | Oily free base, NaCl cell 2970, 1500, 1260, 1230, 1120, 1020 |
| 10 | Compound 10 | 3.7 | 98 | hydrochloride being unable to crystal- " being too hygro- scopic to measure | Oily free base, NaCl cell lize, and fumarate 2970, 1500, 1260, 1225, 1160, 1020 |
| 11 | Compound 11 | 6.1 | 98 | 155—158 | 2920, 2660, 1620, 1460, 1260, 1125 |

TABLE 5

| Example | NMR spectrum ($CDCl_3$, δ: ppm) | Elemental analysis Calcd. (%) Found (%) |
|---|---|---|
| 2 | Oily free base<br>2.14 (s, 6H), 2.23 (s, 3H),<br>1.97—2.77 (m, 4H), 4.77 (d, 1H),<br>4.90 (s, 1H), 6.22 (d, 1H),<br>6.50—7.39 (m, 7H) | $C_{19}H_{23}NOS \cdot HCl$<br>C 65.22 65.20<br>H 6.91 6.84<br>N 4.00 3.89 |
| 3 | Hydrochloride<br>1.22 (t, 6H), 2.20 (s, 3h),<br>2.6—3.4 (m, 9H), 4.89 (d, 1H),<br>5.10 (s, 1H), 6.00 (d, 1H),<br>6.6—7.5 (m, 7H) | $C_{21}H_{27}NOS \cdot HCl$<br>C 66.73 66.59<br>H 7.47 7.49<br>N 3.71 3.58 |
| 4 | Hydrochloride<br>1.27 (t, 6H), 2.6—3.3 (m, 9H),<br>4.83 (d, 1H), 5.13 (s, 1H),<br>6.12 (d, 1H), 6.6—7.6 (m, 7H) | $C_{20}H_{24}ClNOS \cdot HCl$<br>C 60.30 60.03<br>H 6.32 6.17<br>N 3.52 3.66 |
| 5 | Hydrochloride<br>0.9—1.6 (m, 9H), 2.3—3.5 (m,<br>10H), 4.83 (d, 1H), 5.09 (s,<br>1H), 6.07 (d, 1H), 6.6—7.6<br>(m, 7H), 11.9 (br, 1H) | $C_{22}H_{29}NOS \cdot HCl$<br>C 67.41 67.37<br>H 7.71 7.80<br>N 3.57 3.39 |
| 6 | Hydrochloride<br>1.27 (t, 6H), 2.5—3.5 (br, 8H),<br>4.82 (d, 1H), 5.17 (s, 1H),<br>6.03 (d, 1H), 6.7—7.6 (m, 7H),<br>11.8 (br, 1H) | $C_{20}H_{24}FNOS \cdot HCl$<br>C 62.90 63.01<br>H 6.60 6.74<br>N 3.67 3.51 |
| 7 | Hydrochloride, $CDCl_3$ + $d_6$DMSO<br>1.17 (t, 3H), 2.4—3.6 (m, 13H),<br>4.87 (d, 1H), 5.25 (s, 1H),<br>6.03 (d, 1H), 6.6—7.6 (m, 7H) | $C_{20}H_{25}NOS \cdot HCl$<br>C 66.00 66.05<br>H 7.20 6.98<br>N 3.85 3.69 |
| 8 | Hydrochloride, $CDCl_3$ + $d_6$DMSO<br>2.4—3.6 (m, 11H), 4.80 (d, 1H),<br>5.29 (s, 1H), 5.97 (d, 1H),<br>6.7—7.6 (m, 7H) | $C_{18}H_{20}FNOS \cdot HCl$<br>C 61.09 61.32<br>H 5.98 6.09<br>N 3.96 4.00 |
| 9 | Oily free base<br>0.91 (d, 12H), 1.20 (t, 3H),<br>2.3—3.2 (m, 8H), 4.80 (d, 1H),<br>4.91 (s, 1H), 6.29 (d, 1H),<br>6.7—7.5 (m, 7H) | $C_{24}H_{33}NOS$<br>C 75.15 75.02<br>H 8.67 8.58<br>N 3.65 3.77 |
| 10 | Oily free base<br>0.93 (d, 12H), 2.47 (s, 4H),<br>2.7—3.2 (m, 2H), 4.80 (d, 1H),<br>4.85 (s, 1H), 6.22 (d, 1H),<br>6.7—7.5 (m, 7H) | $C_{22}H_{28}FNOS$<br>C 70.74 70.49<br>H 7.56 7.31<br>N 3.75 3.84 |
| 11 | Hydrochloride, $CDCl_3$ + $d_6$DMSO<br>2.22 (s, 3H), 2.4—3.3 (m, 11H),<br>4.82 (d, 1H), 5.23 (s, 1H),<br>6.12 (d, 1H), 6.5—7.6 (m, 7H) | $C_{19}H_{23}NOS \cdot HCl$<br>C 65.22 65.08<br>H 6.91 7.03<br>N 4.00 3.88 |

Example 12

Preparation of tablet

A tablet comprising the following components is prepared in a conventional manner.

| Component | |
|---|---|
| Hydrochloride of compound 1 | 30 mg |
| Lactose | 60 mg |
| Potato starch | 30 mg |
| Polyvinyl alcohol | 2 mg |
| Magnesium stearate | 1 mg |
| Tar pigment | q.s. |

Example 13

Preparation of powder

A powder comprising the following components is prepared in a conventional manner.

| Component | |
|---|---|
| Hydrochloride of compound 2 | 30 mg |
| Lactose | 270 mg |

Example 14

Preparation of syrup

A syrup comprising the following components is prepared in a conventional manner.

| Component | |
|---|---|
| Hydrochloride of compound 7 | 300 mg |
| Sucrose | 40 g |
| Methyl para-hydroxybenzoate | 40 mg |
| Propyl para-hydroxybenzoate | 10 mg |
| Strawberry flavour | 0.1 cc |

Water is added to the above components until the total volume is 100 cc.

**Claims**

1. A novel dibenz[b,e]oxepin derivative represented by the following general formula (Ia):

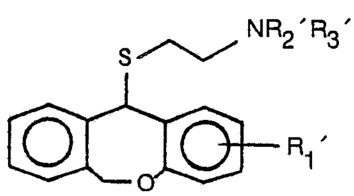

(Ia)

wherein $R_1'$ represents an alkyl group having 1 to 5 carbon atoms or a halogen atom; $R_2'$ and $R_3'$ may be same or different group and each represents an alkyl group having 1 to 5 carbon atoms provided that when $R_2'$ and when $R_3'$ each represents a methyl group, $R_1'$ does not represent a methyl group, a fluorine atom, a chlorine atom or a bromine atom; and the pharmaceutically acceptable acid addition salts thereof.

2. A derivative of claim 1; namely, 2-methyl-11-[2-(diethylamino)ethyl]thio-6,11-dihydro-dibenz[b,e]oxepin.

3. A derivative of claim 1; namely, 4-methyl-11-[2-(diethylamino)ethyl]thio-6,11-dihydro-dibenz[b,e]oxepin.

4. A derivative of claim 1; namely, 2-chloro-11-[2-(diethylamino)ethyl]thio-6,11-dihydro-dibenz[b,e]oxepin.

5. A derivative of claim 1; namely, 2-ethyl-11-[2-(diethylamino)ethyl]thio-6,11-dihydro-dibenz[b,e]oxepin.

6. A derivative of claim 1; namely, 2-fluoro-11-[2-(diethylamino)ethyl]thio-6,11-dihydro-dibenz[b,e]oxepin.

7. A derivative of claim 1; namely, 2-ethyl-11-[2-(dimethylamino)ethyl]thio-6,11-dihydro-dibenz[b,e]oxepin.

8. A derivative of claim 1; namely, 2-ethyl-11-[2-(diisopropylamino)ethyl]thio-6,11-dihydro-dibenz[b,e]oxepin.

9. A derivative of claim 1; namely, 2-fluoro-11-[2-diisopropylamino)ethyl]thio-6,11-dihydrodibenz[b,e]oxepin.

10. A pharmaceutical composition comprising a pharmaceutical carrier and, as an active ingredient, an effective amount of a dibenz[b,e]oxepin derivative represented by the following general formula (Ia):

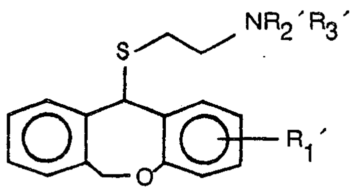

(Ia)

wherein $R_1'$ represents an alkyl group having 1 to 5 carbon atoms or a halogen atom; $R_2'$ and $R_3'$ may be same or different group and each represents an alkyl group having 1 to 5 carbon atoms provided that when $R_2'$ and $R_3'$ each represents a methyl group, $R_1'$ does not represent a methyl group, a fluorine atom, a chlorine atom or a bromine atom; and the pharmaceutically acceptable acid addition salts thereof.

11. A pharmaceutical composition according to claim 10, comprising the 2-methyl-11-[2-(diethylamino)ethyl]thio-6,11-dihydrodibenz[b,e]oxepin.

12. A pharmaceutical composition according to claim 10, comprising the 4-methyl-11-[2-(diethylamino)ethyl]thio-6,11-dihydrodibenz[b,e]oxepin.

13. A pharmaceutical composition according to claim 10, comprising the 2-chloro-11-[2-(diethylamino)ethyl]thio-6,11-dihydrodibenz[b,e]oxepin.

14. A pharmaceutical composition according to claim 10, comprising the 2-ethyl-11-[2-(diethylamino)ethyl]thio-6,11-dihydrodibenz[b,e]oxepin.

15. A pharmaceutical composition according to claim 10, comprising the 2-fluoro-11-[2-(diethylamino)ethyl]thio-6,11-dihydrodibenz[b,e]oxepin.

16. A pharmaceutical composition according to claim 10, comprising the 2-ethyl-11-[2-(dimethylamino)ethyl]thio-6,11-dihydrodibenz[b,e]oxepin.

17. A pharmaceutical composition according to claim 10, comprising the 2-ethyl-11-[2-diisopropylamino)ethyl]thio-6,11-dihydrodibenz[b,e]oxepin.

18. A pharmaceutical composition according to claim 10, comprising the 2-fluoro-11-[2-diisopropylamino)ethyl]thio-6,11-dihydrodibenz[b,e]oxepin.

19. use of a dibenz[b,e]oxepin derivative represented by the following general formula (I)

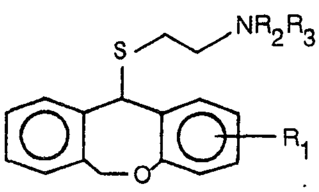

(I)

wherein $R_1$ represents an alkyl group having 1 to 5 carbon atoms or a halogen atom, $R_2$ and $R_3$ may be same or different group and each represent an alkyl group having 1 to 5 carbon atoms, or a pharmaceutically acceptable acid addition salt thereof, for the manufacture of an antiasthmatic medicament.

20. Use of a dibenz[b,e]oxepin derivative according to claim 19, wherein said dibenz[b,e]oxepin derivative is 2-methyl-11-[2-(dimethylamino)ethyl]thio-6,11-dihydrodibenz[b,e]oxepin.

21. Use of a dibenz[b,e]oxepin derivative according to claim 19, wherein said dibenz[b,e]oxepin derivative is 2-fluoro-11-[2-(dimethylamino)ethyl]thio-6,11-dihydrodibenz[b,e]oxepin.

22. Use of a dibenz[b,e]oxepin derivative according to claim 19, wherein said dibenz[b,e]oxepin derivative is 3-methyl-11-[2-(dimethylamino)ethyl]thio-6,11-dihydrodibenz[b,e]oxepin.

**Patentansprüche**

1. Ein neuartiges Dibenz[b,e]oxepin-Derivat der nachstehenden allgemeinen Formel (Ia):

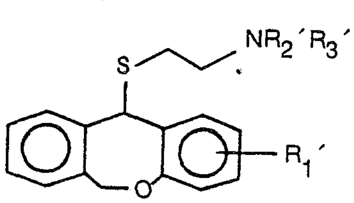

(Ia)

in der $R_1'$ eine Alkylgruppe mit 1 bis 5 Kohlenstoffatomen bzw. ein Halogenatom bedeutet, $R_2'$ und $R_3'$ die gleiche oder eine andere Gruppe sein kann und jeweils eine Alkylgruppe mit 1 bis 5 Kohlenstoffatomen darstellt, mit der Maßgabe daß $R_1'$, wenn sowohl $R_2'$ als auch $R_3'$ eine Methylgruppe ist, weder eine Methylgruppe, ein Fluoratom, ein Chloratom noch ein Bromatom ist; und die pharmazeutisch unbedenklichen sauren Additionssalze desselben.

2. Ein Derivat der Verbindung gemäß Anspruch 1, und zwar 2-Methyl-11-[2-(diethylamino)ethyl]thio-6,11-dihydrodibenz[b,e]oxepin.

3. Ein Derivat der Verbindung gemäß Anspruch 1, und zwar 4-Methyl-11-[2-(diethylamino)ethyl]thio-6,11-dihydrodibenz[b,e]oxepin.

4. Ein Derivat der Verbindung gemäß Anspruch 1, und zwar 2-Chlor-11-[2-(diethylamino)ethyl]thio-6,11-dihydrodibenz[b,e]oxepin.

5. Ein Derivat der Verbindung gemäß Anspruch 1, und zwar 2-Ethyl-11-[2-(diethylamino)ethyl]thio-6,11-dihydrodibenz[b,e]oxepin.

6. Ein Derivat der Verbindung gemäß Anspruch 1, und zwar 2-Fluor-11-[2-(diethylamino)ethyl]thio-6,11-dihydrodibenz[b,e]oxepin.

7. Ein Derivat der Verbindung gemäß Anspruch 1, und zwar 2-Ethyl-11-[2-(dimethylamino)ethyl]thio-6,11-dihydrodibenz[b,e]oxepin.

8. Ein Derivat der Verbindung gemäß Anspruch 1, und zwar 2-Ethyl-11-[2-(diisopropylamino)ethyl]thio-6,11-dihydrodibenz[b,e]oxepin.

9. Ein Derivat der Verbindung gemäß Anspruch 1, und zwar 2-Fluor-11-[2-(diisopropylamino)ethyl]thio-6,11-dihydrodibenz[b,e]oxepin.

10. Eine pharmazeutische Zusammensetzung enthaltend eine pharmazeutische Trägersubstanz und als Wirkstoff einen wirksamen Anteil eines Dibenz[b,e]oxepin-Derivats der nachstehenden allgemeinen Formel (Ia):

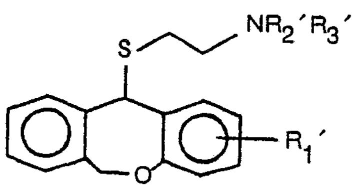

(Ia)

in der $R_1'$ eine Alkylgruppe mit 1 bis 5 Kohlenstoffatomen bzw. einem Halogen bedeutet, $R_2'$ und $R_3'$ die gleiche oder eine andere Gruppe sein kann und jeweils eine Alkylgruppe mit 1 bis 5 Kohlenstoffatomen darstellt, mit der Maßgabe daß $R_1'$, wenn sowohl $R_2'$ als auch $R_3'$ eine Methylgruppe ist, weder eine Methylgruppe, ein Fluoratom, ein Chloratom noch ein Bromatom ist; und die pharmazeutisch unbedenklichen sauren Additionssalze desselben.

11. Eine pharmazeutische Zusammensetzung gemäß Anspruch 10, enthaltend das 2-Methyl-11-[2-(dimethylamino)ethyl]thio-6,11-dihydrodibenz[b,e]oxepin.

12. Eine pharmazeutische Zusammensetzung gemäß Anspruch 10, enthaltend das 4-Methyl-11-[2-(diethylamino)ethyl]thio-6,11-dihydrodibenz[b,e]oxepin.

13. Eine pharmazeutische Zusammensetzung gemäß Anspruch 10, enthaltend das 2-Chlor-11-[2-(diethylamino)ethyl]thio-6,11-dihydrodibenz[b,e]oxepin.

14. Eine pharmazeutische Zusammensetzung gemäß Anspruch 10, enthaltend das 2-Ethyl-11-[2-(diethylamino)ethyl]thio-6,11-dihydrodibenz[b,e]oxepin.

15. Eine pharmazeutische Zusammensetzung gemäß Anspruch 10, enthaltend das 2-Fluor-11-[2-(diethylamino)ethyl]thio-6,11-dihydrodibenz[b,e]oxepin.

16. Eine pharmazeutische Zusammensetzung gemäß Anspruch 10, enthaltend das 2-Ethyl-11-[2-(dimethylamino)ethyl]thio-6,11-dihydrodibenz[b,e]oxepin.

17. Eine pharmazeutische Zusammensetzung gemäß Anspruch 10, enthaltend das 2-Ethyl-11-[2-(diisopropylamino)ethyl]thio-6,11-dihydrodibenz[b,e]oxepin.

18. Eine pharmazeutische Zusammensetzung gemäß Anspruch 10, enthaltend das 2-Fluor-11-[2-(diisopropylamino)ethyl]thio-6,11-dihydrodibenz[b,e]oxepin.

19. Einsatz eines Dibenz[b,e]oxepin-Derivats, dargestellt durch die nachstehende allgemeine Formel (I):

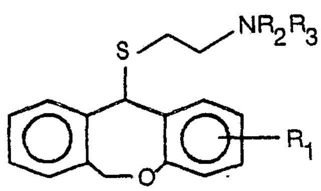

(I)

in der $R_1$ eine Alkylgruppe mit 1 bis 3 Kohlenstoffatomen bzw. ein Halogenatom bedeutet, $R_2$ und $R_3$ die gleiche oder eine andere Gruppe sein kann und jeweils eine Alkylgruppe mit 1 bis 5 Kohlenstoffatomen

darstellt bzw. ein pharmazeutisch unbedenkliches saures Additionssalz desselben zur Herstellung eines Antiasthmaticums.

20. Einsatz eines Dibenz[b,e]oxepin-Derivats gemäß Anspruch 19, wobei es sich bei dem genannten Dibenz[b,e]oxepin-Derivat um 2-Methyl-11-[2-(dimethylamino)ethyl]thio-6,11-dihydrodibenz[b,e]oxepin handelt.

21. Einsatz eines Dibenz[b,e]oxepin-Derivats gemäß Anspruch 19, wobei es sich bei dem genannten Dibenz[b,e]oxepin-Derivat um 2-Fluor-11-[2-(dimethylamino)ethyl]thio-6,11-dihydrodibenz[b,e]oxepin handelt.

22. Einsatz eines Dibenz[b,e]oxepin-Derivats gemäß Anspruch 19, wobei es sich bei dem genannten Dibenz[b,e]oxepin-Derivat um 3-Methyl-11-[2-(dimethylamino)ethyl]thio-6,11-dihydrodibenz[b,e]oxepin handelt.

**Revendications**

1. Nouveau dérivé de la dibenz(b,e)oxépine représenté par la formule générale (Ia):

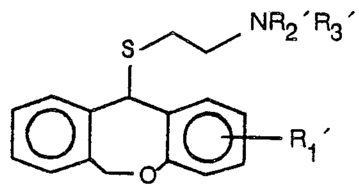

(Ia)

dans laquelle $R_1'$ représente un groupe alkyle ayant de 1 à 5 atomes de carbone ou un atome d'halogène; $R_2'$ et $R_3'$ peuvent être identiques ou différents et représentent chacun un groupe alkyle ayant de 1 à 5 atomes de carbone, à condition que lorsque $R_2'$ et $R_3'$ représentent chacun un groupe méthyle, $R_1'$ ne représente pas un groupe méthyle, un atome de fluor, un atome de chlore ni un atome de brome; et les sels d'addition d'acide de celui-ci, acceptables du point de vue pharmacologique.

2. Dérivé selon la revendication 1, qui est la 2-méthyl-11-[2-(diéthylamino)éthyl]thio-6,11-dihydrodibenz(b,e)oxépine.

3. Dérivé selon la revendication 1, qui est la 4-méthyl-11-[2-(diéthylamino)éthyl]thio-6,11-dihydrodibenz(b,e)oxépine.

4. Dérivé selon la revendication 1, qui est la 2-chloro-11-[2-(diéthylamino)éthyl]thio-6,11-dihydrodibenz(b,e)oxépine.

5. Dérivé selon la revendication 1, qui est la 2-éthyl-11-[2-(diéthylamino)éthyl]thio-6,11-dihydrodibenz(b,e)oxépine.

6. Dérivé selon la revendication 1, qui est la 2-fluoro-11-[2-(diéthylamino)éthyl]thio-6,11-dihydrodibenz(b,e)oxépine.

7. Dérivé selon la revendication 1, qui est la 2-éthyl-11-[2-(diméthylamino)éthyl]thio-6,11-dihydrodibenz(b,e)oxépine.

8. Dérivé selon la revendication 1, qui est la 2-éthyl-11-[2-(diisopropylamino)éthyl]thio-6,11-dihydrodibenz(b,e)oxépine.

9. Dérivé selon la revendication 1, qui est la 2-fluoro-11-[2-(diisopropylamino)éthyl]thio-6,11-dihydrodibenz(b,e)oxépine.

10. Composition pharmaceutique comprenant un véhicule pharmaceutiquement et, en tant qu'ingrédient actif, une quantité efficace d'un dérivé de la dibenz(b,e)oxépine représenté par la formule générale (Ia):

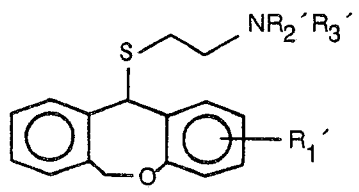

(Ia)

dans laquelle $R_1'$ représente un groupe alkyle ayant de 1 à 5 atomes de carbone ou un atome d'halogène; $R_2'$ et $R_3'$ peuvent être identiques ou différents et représentent chacun un groupe alkyle ayant de 1 à 5 atomes de carbone, à condition que lorsque $R_2'$ et $R_3'$ représentent chacun un groupe méthyle, $R_1'$ ne représente pas un groupe méthyle, un atome de fluor, un atome de chlore ni un atome de brome; et les sels d'addition d'acide de celui-ci, acceptables du point de vue pharmacologique.

11. Composition pharmaceutique selon la revendication 10, comprenant la 2-méthyl-11-[2-(diéthylamino)éthyl]thio-6,11-dihydrodibenz(b,e)oxépine.

12. Composition pharmaceutique selon la revendication 10, comprenant la 4-méthyl-11-[2-(diéthylamino)éthyl]thio-6,11-dihydrodibenz(b,e)oxépine.

13. Composition pharmaceutique selon la revendication 10, comprenant la 2-chloro-11-[2-(diéthylamino)éthyl]thio-6,11-dihydrodibenz(b,e)oxépine.

14. Composition pharmaceutique selon la revendication 10, comprenant la 2-éthyl-11-[2-(diéthylamino)éthyl]thio-6,11-dihydrodibenz(b,e)oxépine.

15. Composition pharmaceutique selon la revendication 10, comprenant la 2-fluoro-11-[2-(diéthylamino)éthyl]thio-6,11-dihydrodibenz(b,e)oxépine.

16. Composition pharmaceutique selon la revendication 10, comprenant la 2-éthyl-11-[2-(diméthylamino)éthyl]thio-6,11-dihydrodibenz(b,e)oxépine.

17. Composition pharmaceutique selon la revendication 10, comprenant la 2-éthyl-11-[2-(diisopropylamino)éthyl]thio-6,11-dihydrodibenz(b,e)oxépine.

18. Composition pharmaceutique selon la revendication 10, comprenant la 2-fluoro-11-[2-(diisopropylamino)éthyl]thio-6,11-dihydrodibenz(b,e)oxépine.

19. Utilisation d'un dérivé de la dibenz(b,e)oxépine représenté par la formule générale (I)

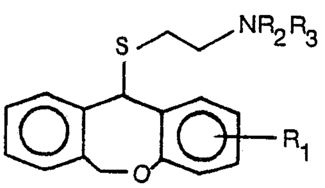

(I)

dans laquelle $R_1$ représente un groupe alkyle ayant de 1 à 5 atomes de carbone ou un atome d'halogène, $R_2$ et $R_3$ peuvent être identiques ou différents et représentent chacun un groupe alkyle ayant de 1 à 5 atomes de carbone, ou un sel d'addition d'acide de celui-ci, acceptable du point de vue pharmacologique, pour la fabrication d'un médicament anti-asthmatique.

20. Utilisation d'un dérivé de la dibenz(b,e)oxépine selon la revendication 19, dans laquelle ledit dérivé de la dibenz(b,e)oxépine est la 2-méthyl-11-[2-(diméthylamino)éthyl]thio-6,11-dihydrodibenz(b,e)oxépine.

21. Utilisation d'un dérivé de la dibenz(b,e)oxépine selon la revendication 19, dans laquelle ledit dérivé de la dibenz(b,e)oxépine est la 2-fluoro-11-[2-(diméthylamino)éthyl]thio-6,11-dihydrodibenz(b,e)oxépine.

22. Utilisation d'un dérivé de la dibenz(b,e)oxépine selon la revendication 19, dans laquelle ledit dérivé de la dibenz(b,e)oxépine est la 3-méthyl-11-[2-(diméthylamino)éthyl]thio-6,11-dihydrodibenz(b,e)oxépine.